# EUROPÄISCHE PATENTSCHRIFT

(11) **EP 1 574 173 B1**
(45) Veröffentlichungstag und Bekanntmachung des Hinweises auf die Patenterteilung: **10.01.2007**
(21) Anmeldenummer: 05002354.8
(22) Anmeldetag: 04.02.2005
(51) Int. Cl.: A61B 17/70

(54) **Elastisches stabförmiges Element für die Anwendung in der Wirbelsäulen- oder Unfallchirurgie und Stabilisierungseinrichtung mit einem solchen stabförmigen Element**
Elastic rod-shaped element for use in spinal or accident surgery and stabilising device comprising such an element
Elément élastique en forme de barre pour la chirurgie de la colonne vertébrale ou d'urgence et appareil de stabilisation comportant un tel élément

(30) Priorität: 09.03.2004 DE 102004011685; 09.03.2004 US 551937
(43) Veröffentlichungstag der Anmeldung: 14.09.2005
(73) Patentinhaber: BIEDERMANN MOTECH GmbH, 78054 VS-Schwenningen (DE)
(72) Erfinder: Harms, Jürgen, 76227 Karlsruhe (DE); Biedermann, Lutz, 78048 VS-Villingen (DE); Rapp, Helmar, 78652 Deisslingen (DE)
(74) Vertreter: Hofer, Dorothea

(56) Entgegenhaltungen:
- EP-A- 1 523 949
- WO-A-03/047442
- GB-A- 2 382 304
- US-A1- 2003 009 226
- US-A1- 2003 220 643

## Beschreibung

Die Erfindung betrifft ein stabförmiges Element für die Anwendung in der Wirbelsäulen- oder Unfallchirurgie und eine Stabilisierungseinrichtung mit einem solchen stabförmigen Element.

Aus der EP 0 669 109 B1 ist eine Stabilisierungseinrichtung zur Stabilisierung benachbarter Rückenwirbel bekannt, die zwei Pedikelschrauben und ein Band umfaßt, welches in den Aufnahmeteilen der Pedikelschrauben jeweils über eine Klammschraube befestigt ist, und welche ein auf das Band aufgezogene Stützelement in Form eines druckfesten Körpers beinhaltet. Diese Stabilisierungseinrichtung ist nicht torsionssteif. Zudem ist die Elastizität hinsichtlich der Biegung mit der Elastizität hinsichtlich Zug- und Druckkräften gekoppelt.

Aus der US 2003/01 09 880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schraube verbindenden Feder und eine solche Feder umfaßt. Die Feder selbst ist als Ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, daß die Feder aufgrund ihrer Elastizität dem Druck der Klemmschraube ausweicht und somit die Fixierung zwischen der Knochenschraube und der Feder gelockert wird. Zudem ist auch hier die Elastizität hinsichtlich der Biegung mit der Elastizität hinsichtlich Zug- und Druckkräften gekoppelt.

Aus der DE 102 36 691 A1 ist eine dynamische Stabilisierungseinrichtung für Knochen, insbesondere für Wirbel bekannt, die wenigstens zwei Knochenverankerungselemente und einen diese verbindenden starren Stab, sowie ein zwischen den Knochenverankerungselementen angeordnetes auf dem Stab vorgesehenes Federelement beinhaltet. Eines der Knochenverankerungselemente ist mit dem Stab so verbunden, daß es in Richtung der Stabachse verschiebbar ist, wobei der Stab einen Anschlag aufweist zur Begrenzung der Bewegung des verschiebbaren Verankerungselements. Diese Stabilisierungseinrichtung erlaubt eine Translationsbewegung in Richtung der Stabachse. Ferner ist aufgrund der Verschiebbarkeit des einen Verankerungselements relativ zum Stab auch eine Rotationsbewegung des Verankerungselements um die Stabachse möglich, eine seitliche Biegung des Stabes jedoch nicht.

Aus der WO 03/047442 ist ein stabförmiges Element nach dem Oberbegriff des Patentanspruchs 1 bekannt.

Die US 2003/0220643 A1 offenbart eine Vorrichtung zum Verhindern der vollen Extension zwischen einem oberen und einem unteren Wirbelkörper. Die Vorrichtung umfasst eine Schraubenfeder, die zwischen zwei Verbindungsabschnitten vorgesehen ist, welche zum Verbinden mit Pedikelschrauben dienen. Die Schraubenfeder kann durch eine Hülse bedeckt sein, die ihrerseits durch ein zweites Federelement umhüllt sein kann.

Bei der dynamischen Stabilisierung von vorgeschädigten Bandscheiben und bei der Verwendung von künstlichen Bandscheiben, insbesondere solchen, die selbst keine Mechanismen für die Bewegungsbegrenzung haben, besteht daher das Bedürfnis der dynamischen Bewegungskontrolle oder Lenkung. Insbesondere sind dazu Stabilisierungseinrichtungen mit elastischem Element, wie z.B. die oben beschriebenen, geeignet, die von der posterioren Seite her eingesetzt werden.

Es ist Aufgabe der Erfindung, ein stabförmiges Element für die Anwendung in der Wirbelsäulen- oder der Unfallchirurgie und eine Stabilisierungseinrichtung mit einem solchen stabförmigen Element bereitzustellen, welches insbesondere geeignet ist, für die dynamische posteriore Stabilisierung oder zur dynamischen Bewegungslenkung bei vorgeschädigter Bandscheibe bzw. bei der Verwendung von künstlichen Bandscheiben und bei dem verschiedene Bewegungsfreiheitsgrade unabhängig voneinander einstellbar sind.

Die Aufgabe wird gelöst durch ein stabförmiges Element nach Patentanspruch 1 bzw. durch eine Stabilisierungseinrichtung nach Patentanspruch 11. Weiterbildungen sind in den Unteransprüchen angegeben.

Die Erfindung weist den Vorteil auf, daß die Elastizität der Verbindung zwischen zwei Knochenverankerungselementen hinsichtlich der Translationsbewegung entkoppelt ist von der Biegeelastizität des die Knochenverankerungselemente verbindenden Stababschnittes. Die Verbindung zwischen den Knochenverankerungselementen mit dem erfindungsgemäßen stabförmigen Element ermöglicht ferner optional eine freie Torsionsbewegung um die Stabachse, wodurch die Kräfte auf die Knochenverankerungselemente reduziert werden können.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Draufsicht auf das stabförmige Element nach einer ersten Ausführungsform;
- Fig. 2: eine Schnittansicht des stabförmigen Elements von Fig. 1;
- Fig. 3: eine schematische Darstellung eines Anwendungsbeispiels des stabförmigen Elements in einem ersten Zustand;
- Fig. 3a: ein Detail aus Fig. 3, schematisch geschnitten;
- Fig. 4: eine schematische Darstellung des Anwendungsbeispiels nach Fig. 3 in einem zweiten Zustand;
- Fig. 5: eine Draufsicht auf das stabförmige Element nach einer zweiten Ausführungsform;
- Fig. 6: eine Schnittansicht des stabförmigen Elements nach Fig. 5 in einer um 90° gedrehten Ansicht; und
- Fig. 7: eine teilgeschnittene Explosionsdarstellung des stabförmigen Elements von Fig. 5 und 6.

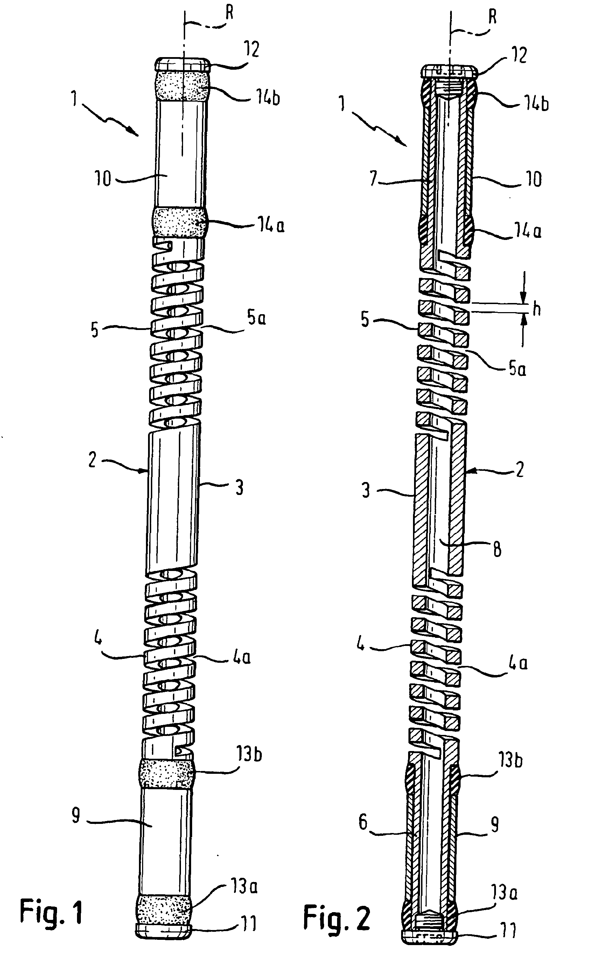

Wie aus Fig. 1 und 2 ersichtlich ist, weist das stabförmige Element 1 nach einer ersten Ausführungsform einen hohlzylindrischen Stab 2 mit einem ersten starren Abschnitt 3 und zu beiden Seiten an diesen angrenzende Federabschnitte 4, 5 sowie an die Federabschnitte jeweils angrenzende starre Endabschnitte 6, 7 auf. Die Federabschnitte 4, 5 haben in diesem Ausführungsbeispiel den selben Außendurchmesser wie der zwischen diesen liegende starre Abschnitt 3, wohingegen die starren Endabschnitte 6, 7 einen kleineren Außendurchmesser haben. Die Federabschnitte 4, 5 sind durch eine in Richtung der Zylinderachse R spiralförmig mit einer vorbestimmten Steigung und über eine vorbestimmte Länge verlaufende und in radialer Richtung in das Innere 8 mündende Ausnehmung 4a bzw. 5a gebildet. Die Länge der Federabschnitte 4, 5 in Richtung der Zylinderachse R, die Höhe h der spiralförmigen Ausnehmung 4a bzw. 5a in Richtung der Zylinderachse, die Steigung der Spirale und der Innendurchmesser des hohlzylindrischen Stabs sind so gewählt, daß die Federabschnitte 4, 5 eine gewünschte Steifigkeit gegenüber axialen Kräften, Biegekräften, d.h. Kräften, die quer zur Stabachse wirken, und Torsionskräften haben.

Auf die starren Endabschnitte 6, 7 ist jeweils eine Hülse 9, 10 aufgeschoben, deren Innendurchmesser gerade so viel größer ist als der Außendurchmesser der starren Endabschnitte 6, 7, daß die Hülse auf den starren Endabschnitten verschiebbar ist. Der Außendurchmesser der Hülse 9 bzw. 10 entspricht dem Außendurchmesser der jeweils angrenzenden Federabschnitte 4 bzw. 5. Die Länge der Hülsen 9, 10 ist kleiner als die Länge der starren Endabschnitte 6, 7.

An den freien Enden der starren Endabschnitte 6, 7 weist der hohlzylindrische Stab 2 ein Innengewinde auf, in das jeweils eine Sicherungsschraube 11, 12 eingeschraubt ist. Die Sicherungsschraube 11, 12 weist jeweils einen Außendurchmesser auf, der größer ist als der Außendurchmesser der Hülse 9, 10.

Zwischen der Hülse 9 und dem Federabschnitt 4a sowie der Sicherungsschraube 11 bzw. zwischen der Hülse 10 und dem Federabschnitt 5, sowie der Sicherungsschraube 12 sind jeweils zwei elastische Ringe 13a, 13b bzw. 14a, 14b vorgesehen, deren Außendurchmesser etwas größer ist als der Außendurchmesser der Hülse 9, 10. In nicht belastetem Zustand schließen die elastischen Ringe 13a, 13b die Hülse 9 und die elastischen Ringe 14a und 14b die Hülse 10 zwischen sich ein, so daß die Hülse nicht hin und her rutschen kann. Die elastischen Ringe sind aus einem körperverträglichen Elastomer gebildet. Die Breite der elastischen Ringe 13a, 13b bzw. 14a, 14b in axialer Richtung ist in Abhängigkeit von der Komprimierbarkeit des Materials der Ringe so gewählt, daß eine vorbestimmte Verschiebung der Hülse 9, 10 durch Zusammendrücken des entsprechenden elastischen Rings möglich ist.

Die Länge des starren Abschnitts 3 und der starren Endabschnitte 6, 7, sowie die Länge der Hülsen 9, 10 in Richtung der Zylinderachse R ist so bemessen, daß sie wenigstens so groß wie der Durchmesser eines nachfolgend beschriebenen Fixierelements ist, welches das stabförmige Element an einem Knochenverankerungselement fixiert.

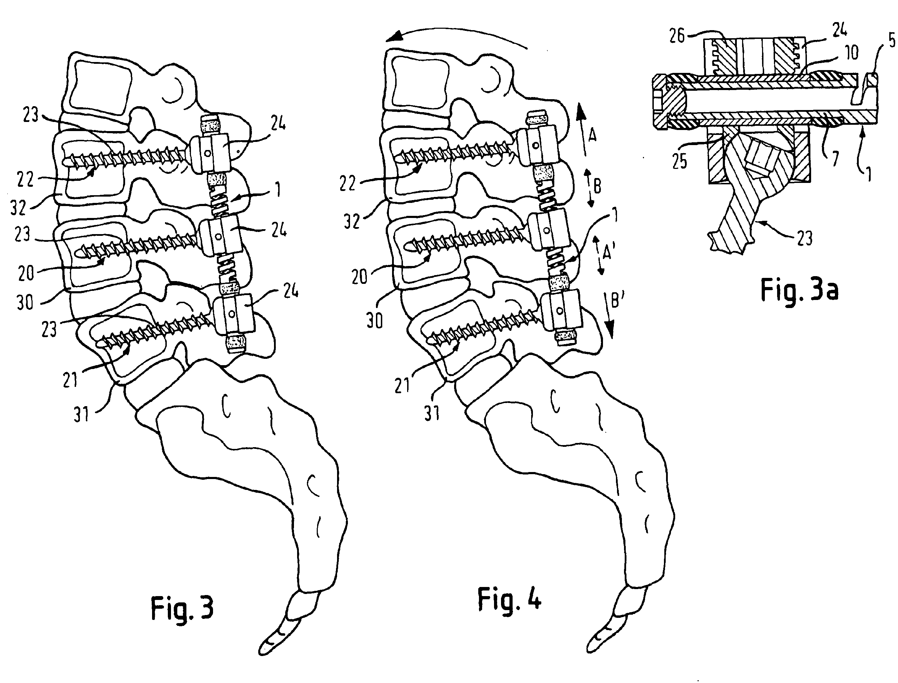

Wie aus Fig. 3 ersichtlich ist, ist das stabförmige Element in einem Anwendungsbeispiel Teil einer Stabilisierungseinrichtung, die eine erste Pedikelschraube 20 umfaßt, die mit dem starren Abschnitt 3 verbunden ist und in einem ersten Wirbel 30 verankert ist, eine zweite Pedikelschraube 21, die mit der Hülse 9 auf dem starren Endabschnitt 6 fest verbunden ist und die in einem an dem Wirbel 30 angrenzenden Wirbel 31 verankert ist, und eine dritte Pedikelschraube 22, die in einem dritten Wirbel 32 angrenzend an den Wirbel 30 verankert ist und die mit der Hülse 10 auf dem starren Endabschnitt 7 fest verbunden ist.

Die Pedikelschrauben 20, 21, 22 sind wie insbesondere in Fig. 3a gezeigt ist, vorzugsweise Polyaxialschrauben, die jeweils ein Schraubenelement 23 und ein damit gelenkig verbundenes Aufnahmeteil 24, sowie ein auf den Kopf des Schraubenelements 23 einwirkendes Druckstück 25 und ein Fixierelement 26 zum Fixieren des stabförmigen Elements in dem Aufnahmeteil 24 aufweisen. In dem dargestellten Ausführungsbeispiel weist das Aufnahmeteil einen Kanal zum Einführen des stabförmigen Elements und eine in das Aufnahmeteil einschraubbare Innenschraube auf. Die Länge des starren Abschnitts 3 und die Länge der Hülsen 9, 10 ist somit wenigstens so groß, wie der Durchmesser der Innenschraube 26, die auf das stabförmige Element jeweils in den entsprechenden Abschnitten drückt.

Im Betrieb werden zunächst die Pedikelschrauben in die Wirbel eingeschraubt und dann das vormontierte, wie in Fig. 1 und 2 dargestellte stabförmige Element in die Aufnahmeteile 24 eingelegt und fixiert. Somit ist die Pedikelschraube 20 des in der Mitte liegenden Wirbels 30 fest mit dem starren Abschnitt 3 des stabförmigen Elements verbunden, während die Pedikelschrauben 21, 22 der jeweils benachbarten Wirbel fest mit den Hülsen 9, 10 verbunden sind.

Wie in Fig. 4 gezeigt ist, bildet bei der gezeigten mehrsegmentalen Stabilisierungseinrichtung die mit dem mittleren starren Abschnitt 3 verbundene Pedikelschraube 20 einen Fixpunkt bei der Bewegung. Bei einer Flexion der Wirbelsäule werden die Federabschnitte 4, 5 gedehnt, wodurch sich der Abstand zwischen den Pedikelschrauben 21 und 22 in axialer Richtung vergrößert. Gleichzeitig drücken die Hülsen 9, 10 gegen den jeweils äußeren, der Sicherungsschrauben 11, 12, benachbarten elastischen Ring 13a bzw. 14b und komprimieren diesen. Die Sicherungsschrauben 11, 12 bilden dabei einen Anschlag für die elastischen Ringe 13a, 14b.

Bei Flexion der Wirbelsäule werden zunächst im wesentlichen nur die äußeren elastischen Ringe 13a, 14b deformiert (durch die Pfeile A, A' in Fig. 4 veranschaulicht), während die Federabschnitte 4, 5 sich nur wenig ausdehnen (durch die Pfeile B, B' in Fig. 4 veranschaulicht). Mit zunehmender Flexion nimmt die rücktreibende Kraft der deformierten äußeren elastischen Ringe 13a, 14b im Vergleich zu der rücktreibenden Kraft der Federabschnitte 4, 5 überproportional zu. So verbleibt schließlich nur noch eine eingeschränkte Beweglichkeit durch die Federabschnitte 4, 5, während sich die äußeren elastischen Ringe 13a, 14b kaum mehr weiter deformieren können.

Extension der Wirbelsäule erfolgt ganz analog gegen die rücktreibenden Kräfte der inneren elastischen Ringe 13b, 14a und die rücktreibende Kraft der Federabschnitte 4, 5.

Eine Rotation der Hülsen um die Zylinderachse R ist möglich. Damit wird verhindert, daß Torsionskräfte um die Zylinderachse auf die Verankerung der Pedikelschrauben wirken und diese lockern.

In einer Abwandlung der ersten Ausführungsform weist das stabförmige Element nur einen Federabschnitt und einen Abschnitt mit Hülse auf. Es ist dann zwischen zwei benachbarten Wirbeln zu verwenden, wobei dann ein Verankerungselement mit dem starren Abschnitt fest verbunden ist, und das zweite Verankerungselement mit der entsprechenden Hülse verbunden ist.

In einer weiteren Abwandlung ist der starre Abschnitt nicht vorgesehen, sondern es sind nur die zwei Endabschnitte mit Hülsen vorgesehen, wobei sich zwischen diesen dann ein Federabschnitt erstreckt.

In einer weiteren Abwandlung sind die Durchmesser der Federabschnitte und des starren Abschnitts bzw. auch der Federabschnitte untereinander unterschiedlich. Auch können die Federabschnitte eine unterschiedliche Elastizität haben. Die Ausbildung des stabförmigen Elements ist nicht auf den in den Fig. 1 bis 4 gezeigten symmetrischen Aufbau beschränkt, sondern kann auch unsymmetrisch ausgebildet sein, indem z.B. unterschiedliche Längen der Abschnitte ausgebildet sind. Anstelle einer Sicherungsschraube kann auch eine andere Art von Endanschlag, wie z.B. ein aufgesetzter Ring oder ähnliches vorgesehen sein.

Die Verankerungselemente können als Monoaxialschrauben oder Polyaxialschrauben oder als Haken in bekannter Weise ausgebildet sein.

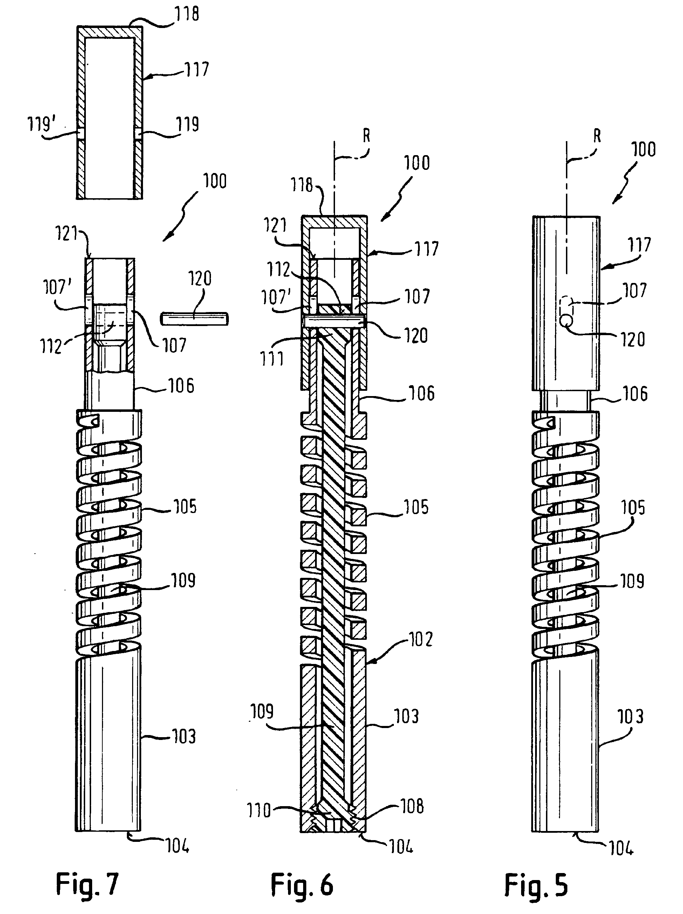

In einer in den Fig. 5 bis 7 gezeigten zweiten Ausführungsform weist das stabförmige Element 100 einen hohlzylindrischen Stab 102 mit einem ersten starren Abschnitt 103 mit einem freien Ende 104, einen an den ersten starren Abschnitt 103 angrenzenden Federabschnitt 105 und einem an den Federabschnitt 105 angrenzenden zweiten starren Abschnitt 106 auf. Der Federabschnitt 106 ist wie bei der ersten Ausführungsform durch eine spiralförmige Ausnehmung in der Wand des hohlzylindrischen Stabs 102 gebildet. Der Federabschnitt 105 und der starre Abschnitt 103 haben den selben Außendurchmesser, wohingegen der zweite starre Abschnitt 106 einen geringeren Außendurchmesser aufweist ähnlich wie der starre Endabschnitt 6 bei der ersten Ausführungsform.

Wie insbesondere aus den Fig. 6 und 7 ersichtlich ist, weist der starre Endabschnitt 106 in einem vorbestimmten Abstand von einem freien Ende 2 um 180° versetzte Langlöcher 107, 107' auf. Angrenzend an ein freies Ende weist der starre Abschnitt 103 ein Innengewinde 108 auf.

In dem hohlzylindrischen Stab 102 ist ein Federelement 109 vorgesehen, was im wesentlichen stabförmig ausgebildet ist und an seinem einen Ende einen ersten Verbindungsabschnitt 110 aufweist mit einem Außengewinde, welches mit dem Innengewinde 108 des starren Abschnitts 103 zusammenwirkt. An seinem dem ersten Verbindungsabschnitt gegenüberliegenden Ende weist das Federelement 109 einen zweiten Verbindungsabschnitt 111 auf, der, wie insbesondere aus den Fig. 6 und 7 ersichtlich ist, mit einer durchgehenden Bohrung 112 in radialer Richtung versehen ist. Die Länge des Federelements 109 ist so, daß in einem Zustand, in dem das Federelement 109 am Ende des starren Abschnitts 103 befestigt ist und in Richtung der Zylinderachse auf ein vorbestimmtes Maß gedehnt ist, die radiale Bohrung 112 mit den Langlöchern 107, 107' zusammenfällt bzw. durch diese überdeckt wird.

Es ist ferner eine Hülse 117 vorgesehen, deren Innendurchmesser um so viel größer ist als der Außendurchmesser des starren Endabschnitts 106, daß die Hülse 117 auf dem starren Endabschnitt eine Gleitbewegung ausführen kann. Der Außendurchmesser der Hülse 117 entspricht dem Außendurchmesser des Federabschnitts 105 bzw. des starren Abschnitts 103. Die Hülse ist an ihrem in montiertem Zustand dem Federabschnitt abgewandten Ende mit einer Deckfläche 118 geschlossen.

Die Hülse 117 weist ferner in ihrer Außenwand und in einem Abstand von ihrer Deckfläche 118 in ihrer Wandung zwei um 180° versetzte kreisförmige Öffnungen 119, 119' auf, deren Durchmesser dem Durchmesser der Bohrung 112 des Federelements 109 entspricht.

In montiertem Zustand ist durch die Öffnungen 119, 119' der Hülse 117, sowie durch die Langlöcher 107, 107' des starren Endabschnitts 106 des Stabs, sowie durch die Bohrung 112 des Federelements 109 ein Stift 120 hindurchgeführt, der in den Öffnungen 119, 119' und in der Bohrung 112 paßförmig liegt. Die Länge des Stifts 120 ist gleich oder etwas kleiner als der Außendurchmesser der Hülse 117.

Die Abmessungen des starren Abschnitts 106 des Stabs, die Position der Langlöcher 107, 107', die Länge der Hülse 117 und die Position der Öffnungen 119, 119' sind so gewählt, daß in montiertem Zustand der starre Abschnitt 106 in der Hülse 117 um eine durch die Länge des Langlochs definierte Strecke gleiten kann, die durch das Anstoßen des Stifts 120 an dem Langloch begrenzt ist.

Das Federelement ist bevorzugt aus einem elastischen Kunststoffmaterial ausgebildet, wobei die Endabschnitte 110, 111 zum Verbinden mit dem Ende des starren Abschnitts bzw. mit dem Stift bevorzugt starr ausgebildet sind. Der übrige Teil des stabförmigen Elements ist aus einem körperfreundlichen Metall wie beispielsweise Titan oder aus einem köperfreundlichen Kunststoff gebildet.

Im Betrieb wird der starre Abschnitt 103 mit einem im Knochen verankerten Verankerungselement, beispielsweise mit einer der zuvor beschriebenen Polyaxialschrauben verbunden, während das durch die Hülse 117 andere Ende des stabförmigen Elements mit einem zweiten Knochenverankerungselement verbunden wird. Bei einer Dehnung des stabförmigen Elements 100 aus dem relaxierten Zustand kommt es zunächst im wesentlichen nur zu einer Verschiebung der Hülse 117 relativ zu dem starren Abschnitt 106, während sich der Federabschnitt 105 aufgrund seiner gegenüber dem Federelement 109 geringeren Elastizität kaum deformiert. Schließlich erreicht der Stift 120 das eine Ende der Langlöcher 107, 107', wodurch eine weitere Verschiebung der Hülse 117 relativ zu dem starren Abschnitt 106 verhindert wird und eine weitere Dehnung des stabförmigen Elements dann nur noch gegen die Federkraft des Federabschnitts 105 erfolgen kann. Die Kompression des stabförmigen Elementes erfolgt ganz analog zunächst bis zum Anschlag des Stiftes 120 am anderen Ende der Langlöcher 107, 107' gegen die rücktreibende Kraft des Federelementes 109 durch Verschiebung der Hülse 117 relativ zu dem starren Abschnitt 106. Nach dem Anschlag des Stiftes erfolgt die Kompression des stabförmigen Elementes gegen die rücktreibende Kraft des Federabschnittes 105.

Die Elastizität des stabförmigen Elementes hinsichtlich Biegung, d.h. Einwirkung von Kräften quer zur Stabachse, ist allein durch die Biegeelastizität des Federabschnittes 105 gegeben.

In der zweiten Ausführungsform übernimmt somit das Federelement 109 die Funktion der elastischen Ringe 13a, 13b, 14a, 14b in der ersten Ausführungsform, während der Federabschnitt 105 den Federabschnitten 4, 5 entspricht.

Im Unterschied zur ersten Ausführungsform ist jedoch bei dem stabförmigen Element 100 nach der zweiten Ausführungsform eine Rotation um die Zylinderachse R nicht möglich, da diese durch den in den Langlöchern 107, 107' geführten Stift 112 verhindert wird.

In einer Abwandlung der zweiten Ausführungsform ist das stabförmige Element wie bei der ersten Ausführungsform für die mehrsegmentare Anwendung ausgebildet. Hierzu schließt sich an das freie Ende 104 gemäß Fig. 6 ein weiterer Federabschnitt mit einer weiteren Hülse mit symmetrischem Aufbau an. Entweder ist hierzu ein zweites Federelement vorgesehen, oder das gezeigte Federelement ist von größerer Länge und in seiner Mitte auf irgendeine Art mit dem starren Abschnitt 103 verbunden, oder das Federelement ist größerer Länge und nicht mit dem starren Endabschnitt verbunden, sondern nur hindurchgeführt.

Auch bei dieser Ausführungsform müssen die Längen und Durchmesser nicht wie beschrieben ausgebildet sein, sondern die einzelnen Abschnitte können unterschiedliche Längen und/oder Durchmesser aufweisen.

Ferner müssen die stabförmigen Elemente bzw. Hülsen noch der ersten und zweiten Ausführungsform keinen runden Querschnitt haben, sondern können z.B. auch oval oder anders ausgebildet sein. Durch einen nicht-kreisförmigen Querschnitt der starren Abschnitte 6, 7 und der Hülsen 9, 10 läßt sich so eine Blockierung der Torsionsbewegung realisieren.

In einer weiteren Abwandlung ist das Federelement 109 aus einem Metall oder einem körperverträglichen Kunststoff als Schraubenfeder ausgebildet.

Die Elemente der vorgenannten Ausführungsformen können miteinander kombiniert werden.

## Patentansprüche

1. Stabförmiges Element für die Anwendung in der Wirbelsäulen- oder Unfallchirurgie mit
einem ersten Abschnitt (3; 103) zum Verbinden mit einem ersten Knochenverankerungselement (20; 21)
und einem zweiten Abschnitt (6; 7; 106) zum Verbinden mit einem zweiten Knochenverankerungselement (22; 21),
sowie mit einem zwischen dem ersten und dem zweiten Abschnitt angeordneten ersten elastischen Element (4; 5; 105) und mit einem zweiten elastischen Element (13a, 13b; 14a, 14b; 109), **dadurch gekennzeichnet, dass** das erste elastische Element so ausgebildet ist, daß es bei einer Krafteinwirkung quer zur Stabachse elastisch deformierbar ist, und
wobei eine auf dem zweiten Abschnitt (6; 7; 106) in Richtung der Stabachse verschiebbar geführte und mit dem zweiten Knochenverankerungselement verbindbare Hülse (9; 10; 117) vorgesehen ist und
wobei das zweite elastische Element (13a, 13b; 14a, 14b; 109) zum Dämpfen der axialen Verschiebebewegung der Hülse zu dem zweiten Abschnitt vorgesehen ist.

2. Stabförmiges Element nach Anspruch 1, **dadurch gekennzeichnet, daß** ein Anschlag zum Begrenzen der Verschiebebewegung vorgesehen ist.

3. Stabförmiges Element nach Anspruch 1 oder 2, **dadurch gekennzeichnet, daß** das erste elastische Element (4; 5; 105) bei Krafteinwirkung quer und in Richtung der Stabachse elastisch deformierbar ist.

4. Element nach einem der Ansprüche 1 bis 3, **dadurch gekennzeichnet, daß** das erste elastische Element (4; 5; 105) im wesentlichen zylindrisch ausgebildet ist und mit jeweils einem starren Abschnitt (3; 6; 7; 103; 106) an seinen Stirnseiten verbunden ist.

5. Stabförmiges Element nach einem der Ansprüche 1 bis 4, **dadurch gekennzeichnet, daß** das zweite elastische Element ein Dämpfungsring (13a, 13b; 14a, 14b) aus einem elastischen Material ist.

6. Stabförmiges Element nach Anspruch 4 oder 5, **dadurch gekennzeichnet, daß** an wenigstens einer Seite der Hülse ein Dämpfungsring (13a, 13b; 14a, 14b) aus einem elastischen Material vorgesehen ist.

7. Stabförmiges Element nach einem der Ansprüche 1 bis 5, **dadurch gekennzeichnet, daß** das zweite elastische Element eine mit dem ersten Abschnitt und dem zweiten Abschnitt verbundene Feder (109), die sich in axialer Richtung erstreckt, beinhaltet.

8. Stabförmiges Element nach einem der Ansprüche 1 bis 7, **dadurch gekennzeichnet, daß** das erste elastische Element (4, 5; 105) und das zweite elastische Element (13a, 13b; 14a, 14b; 109) unterschiedliche Federkonstanten in axialer Richtung des Stabs haben.

9. Stabförmiges Element nach einem der Ansprüche 1 bis 8, **dadurch gekennzeichnet, daß** der erste Abschnitt (3; 103) und der zweite Abschnitt (6; 7; 106) in Umfangsrichtung gegeneinander verschiebbar sind.

10. Stabförmiges Element nach einem der Ansprüche 1 bis 9, **dadurch gekennzeichnet, daß** der erste Abschnitt (103) und der zweite Abschnitt (106) in Umfangsrichtung gegen Verdrehen gesichert sind.

11. Stabilisierungseinrichtung mit einem ersten Knochenverankerungselement und einem zweiten Knochenverankerungselement und einem stabförmigen Element nach einem der Ansprüche 1 bis 10.

## Claims

1. Rod-shaped element for use in spinal or trauma surgery, with
a first section (3; 103) for connecting to a first bone anchoring element (20; 21),
and a second section (6; 7; 106) for connecting to a second bone anchoring element (22; 21),
as well as with a first elastic element (4; 5; 105) arranged between the first and second sections and with a second elastic element (13a, 13b; 14a, 14b; 109);
**characterized in that** the first elastic element is formed such that it is elastically deformable upon force acting transverse to the rod axis, and
wherein a sleeve (9; 10; 117) movably guided on the second section (6; 7; 106) in the direction of the rod axis and connectable to the second bone anchoring element is provided, and
wherein the second elastic element (13a, 13b; 14a, 14b; 109) is provided for damping the axial shifting motion of the sleeve relative to the second section.

2. Rod-shaped element according to Claim 1, **characterized in that** a stop for limiting the shifting motion is provided.

3. Rod-shaped element according to Claim 1 or 2, **characterized in that** the first elastic element (4; 5; 105) is elastically deformable upon force acting transverse to and in the direction of the rod axis.

4. Element according to any one of the Claims 1 to 3, **characterized in that** the first elastic element (4; 5; 105) is essentially cylindrically formed and connected to a rigid section (3; 6; 7; 103; 106) each on its front faces.

5. Rod-shaped element according to one of Claims 1 to 4, **characterized in that** the second elastic element is a damping ring (13a, 13b; 14a, 14b) made of an elastic material.

6. Rod-shaped element according to Claim 4 or 5, **characterized in that** at least on one side of the sleeve a damping ring (13a, 13b; 14a, 14b) made of an elastic material is provided.

7. Rod-shaped element according to one of Claims 1 to 5, **characterized in that** the second elastic element includes a spring (109) connected to the first section and to the second section which extends in the axial direction.

8. Rod-shaped element according to any one of the Claims 1 to 7, **characterized in that** the first elastic element (4, 5; 105) and the second elastic element (13a, 13b; 14a, 14b; 109) have different spring constants in the axial direction of the rod.

9. Rod-shaped element according to any one of the Claims 1 to 8, **characterized in that** the first section (3; 103) and the second section (6; 7; 106) are movable relative to each other in the circumferential direction.

10. Rod-shaped element according to any one of the Claims 1 to 9, **characterized in that** the first section (103) and the second section (106) are secured against rotation in the circumferential direction.

11. Stabilization device with a first bone anchoring element and a second bone anchoring element and a rod-shaped element according to any one of the Claims 1 to 10.

## Revendications

1. Elément en forme de barre pour l'application dans la chirurgie de la colonne vertébrale ou la chirurgie de traumatologie comprenant une première partie (3 ; 103) pour la liaison avec un premier élément d'ancrage osseux (20 ; 21) et une seconde partie (6 ; 7 ; 106) pour la liaison avec un deuxième élément d'ancrage osseux (22 ; 21), et avec un premier élément élastique (4 ; 5 ; 105) disposé entre la première et la seconde partie et avec un second élément (13a, 13b ; 14a, 14b ; 109) élastique, **caractérisé en ce que** le premier élément élastique est réalisé de telle sorte qu'il peut être déformé élastiquement transversalement à l'axe de la barre lors d'un effet de force et sur lequel un manchon (9 ; 10 ; 117) guidé sur la seconde partie (6 ; 7 ; 106) de façon coulissante en direction de l'axe de la barre et pouvant être relié au second élément d'ancrage osseux et dans lequel le second élément (13a, 13b ; 14a, 14b ; 109) élastique est prévu pour l'amortissement du mouvement de coulissement axial du manchon vers la seconde partie.

2. Elément en forme de barre selon la revendication 1, **caractérisé en ce qu'**une butée est prévue pour la délimitation du mouvement de coulissement.

3. Elément en forme de barre selon la revendication 1 ou 2, **caractérisé en ce que** le premier élément (4 ; 5 ; 105) élastique peut être déformé élastiquement sous l'effet d'une force transversalement et en direction de l'axe de la barre.

4. Elément selon l'une quelconque des revendications 1 à 3, **caractérisé en ce que** le premier élément (4 ; 5 ; 105) élastique est conçu de façon sensiblement cylindrique et est relié par respectivement une partie (3 ; 6 ; 7 ; 103 ; 106) rigide sur ses côtés avant.

5. Elément en forme de barre selon l'une quelconque des revendications 1 à 4, **caractérisé en ce que** le second élément élastique est une bague d'amortissement (13a, 13b ; 14a, 14b) à base d'un matériau élastique.

6. Elément en forme de barre selon la revendication 4 ou 5, **caractérisé en ce qu'**une bague d'amortissement (13a, 13b ; 14a, 14b) à base d'un matériau élastique est prévue sur au moins un côté du manchon.

7. Elément en forme de barre selon l'une quelconque des revendications 1 à 5, **caractérisé en ce que** le second élément élastique contient un ressort (109) relié à la première partie et à la seconde partie qui s'étend dans le sens axial.

8. Elément en forme de barre selon l'une quelconque des revendications 1 à 7, **caractérisé en ce que** le premier élément élastique (4, 5 ; 105) et le second élément élastique (13a, 13b ; 14a, 14b ; 109) ont différentes constantes de ressort dans la direction axiale de la barre.

9. Elément en forme de barre selon l'une quelconque des revendications 1 à 8, **caractérisé en ce que** la première partie (3 ; 103) et la seconde partie (6 ; 7 ; 106) peuvent coulisser l'une par rapport à l'autre dans la direction périphérique.

10. Elément en forme de barre selon l'une quelconque des revendications 1 à 9, **caractérisé en ce que** la première partie (103) et la seconde partie (106) sont bloquées dans le sens périphérique vis-à-vis d'une rotation.

11. Dispositif de stabilisation comprenant un premier élément d'ancrage osseux et un second élément d'ancrage osseux et un élément en forme de barre selon l'une quelconque des revendications 1 à 10.
